# EUROPEAN PATENT APPLICATION

(11) **EP 2 418 275 A1**
(43) Date of publication of application: **15.02.2012**
(21) Application number: 10761222.8
(22) Date of filing: 08.04.2010
(51) Int. Cl.: C12N 9/40, C12N 9/42, C12N 15/81

(54) **STRAINS OF S.CEREVISIAE, CAPABLE OF GROWING IN MEDIA WITH MELIBIOSE, STACHYOSE AND RAFFINOSE**

(30) Priority: 08.04.2009 ES 200900961
(71) Applicant: Universidade Da Coruña, 15001 A Coruña (ES)
(72) Inventor: BECERRA FERNÁNDEZ, Manuel, E-15071 A Coruña (ES); CERDÁN VILLANUEVA, María Esperanza, E-15071 A Coruña (ES); FERNÁNDEZ LEIRO, Rafael, E-15071 A Coruña (ES); GONZÁLEZ SISO, María Isabel, E-15071 A Coruña (ES); PEREIRA RODRÍGUEZ, Ángel, E-15071 A Coruña (ES)
(74) Representative: ABG Patentes, S.L.
(86) International application number: PCT/ES2010/070219
(87) International publication number: WO 2010/116020

(57) **Abstract**

The invention relates to strains of S. *cerevisiae* capable of secreting α-galactosidase into culture medium, as well as to methods for obtaining α-galactosidase using said strains and methods for producing biomass and bioethanol by means of culturing said strains in media rich in galactose. In another aspect, the invention provides methods for expressing recombinant proteins using a composition rich in galactose as a culture medium for the microorganisms producing said protein.

SEQUENCE LISTING

<110>  UNIVERSIDADE DA CORUÑA

<120>  STRAINS OF S.CEREVISIAE CAPABLE OF GROWING IN MEDIA WITH MELIBIOSE,
STACHYOSE AND RAFFINOSE

<130>  P4444EPPC

<140>  EP 10761222.8
<141>  2010-04-08

<150>   ES P200900961
<151>   2009-04-08

<160>  10

<170>  PatentIn version 3.5

<210>  1
<211>  1201
<212>  DNA
<213>  Saccharomyces cerevisiae

<400>  1
gatccttcaa tatgcgcaca tacgctgtta tgttcaaggt cccttcgttt aagaacgaaa      60
gcggtcttcc ttttgaggga tgtttcaagt tgttcaaatc tatcaaattt gcaaatcccc     120
agtctgtatc tagagcgttg aatcggtgat gcgatttgtt aattaaattg atggtgtcac     180
cattaccagg tctagatata ccaatggcaa actgagcaca acaataccag tccggatcaa     240
ctggcaccat ctctcccgta gtctcatcta atttttcttc cggatgaggt tccagatata     300
ccgcaacacc tttattatgg tttccctgag ggaataatag aatgtcccat tcgaaatcac     360
caattctaaa cctgggcgaa ttgtatttcg ggtttgttaa ctcgttccag tcaggaatgt     420
tccacgtgaa gctatcttcc agcaaagtct ccacttcttc atcaaattgt gggagaatac     480
tcccaatgct cttatctatg ggacttccgg gaaacacagt accgatactt cccaattcgt     540
cttcagagct cattgtttgt ttgaagagac taatcaaaga atcgttttct caaaaaaatt     600
aatatcttaa ctgatagttt gatcaaaggg gcaaaacgta ggggcaaaca aacggaaaaa     660
tcgtttctca aattttctga tgccaagaac tctaaccagt cttatctaaa aattgcctta     720
tgatccgtct ctccggttac agcctgtgta actgattaat cctgcctttc taatcaccat     780
tctaatgttt taattaaggg attttgtctt cattaacggc tttcgctcat aaaaatgtta     840
tgacgttttg cccgcaggcg ggaaaccatc cacttcacga gactgatctc ctctgccgga     900
acaccgggca tctccaactt ataagttgga gaaataagag aatttcagat tgagagaatg     960
aaaaaaaaaa aaaaaaaaaa ggcagaggag agcatagaaa tggggttcac tttttggtaa    1020
agctatagca tgcctatcac atataaatag agtgccagta gcgacttttt tcacactcga    1080
aatactctta ctactgctct cttgttgttt ttatcacttc ttgtttcttc ttggtaaata    1140
gaatatcaag ctacaaaaag catacaatca actatcaact attaactata tcgtaataca    1200
c                                                                    1201


<210>  2
<211>  24
<212>  DNA
<213>  Artificial sequence

<220>
<223>  Sequence encoding tag peptide for immunological detection

<400>  2
gactacaagg atgacgatga caag                                             24


<210>  3
<211>  249
<212>  DNA
<213>  Saccharomyces cerevisiae

<400>  3
atgagatttc cttcaatttt tactgcagtt ttattcgcag catcctccgc attagctgct      60
ccagtcaaca ctacaacaga agatgaaacg gcacaaattc cggctgaagc tgtcatcggt     120
tacttagatt tagaagggga tttcgatgtt gctgttttgc cattttccaa cagcacaaat     180
aacgggttat tgtttataaa tactactatt gccagcattg ctgctaaaga agaaggggta     240
tctttggat                                                             249


<210>  4
<211>  1458
<212>  DNA
<213>  Saccharomyces cerevisiae

<400>  4
atgtttgctt tctactttct caccgcatgc atcagtttga agggcgtttt tggggtgtct      60
ccgagttaca atggccttgg tctcactcca cagatgggtt gggacaactg gaatacgttt     120
gcctgcgatg tcagtgaaca gctacttcta gacaccgctg atagaatttc tgacttgggg     180
ctaaaggata tgggttacaa gtatatcatt ctggatgact gctggtctag cggcagagat     240
tccgacggtt tcctcgttgc agatgaacaa aaatttccca atggtatggg ccatgttgca     300
gaccacctgc ataataacag ctttcttttc ggtatgtatt cgtctgctgg tgagtacacc     360
tgtgctggat atcctgggtc tctgggtcgt gaggaagaag atgcacagtt ctttgcaaat     420
aaccgcgttg actacttgaa gtacgataat tgttacaata agggtcagtt tggtacaccg     480
gaaatttctt accaccgtta caaggccatg tcagatgctt tgaataaaac tggtaggcct     540
atattctatt ctctatgtaa ctggggtcag gatttaacat tttactgggg ctctggtatc     600
gccaattctt ggagaatgag tggagatgtt actgctgagt tcactcgtcc agatagcaga     660
tgtccctgtg atggcgatga atacgattgc aagtacgccg gtttccattg ttctattatg     720
aatattctta acaaggcagc tccaatgggg caaaatgcag gtgttggtgg ttggaatgat     780
ctggacaatc tagaggttgg tgtcgggaat ttgactgacg atgaggaaaa ggcacatttc     840
tctatgtggg caatggtaaa gtctccactt atcattggtg ccaatgtgaa taacttaaag     900
gcatcttcgt actcaatcta tagtcaagcc tctgtcatcg caattaatca agattcaaat     960
ggtattccag caacaagagt ctggagatat tatgtttcag acacagatga atatggacaa    1020
ggtgaaattc aaatgtggag tggtcctctt gacaatggtg atcaagtggt tgctttattg    1080
aatggaggaa gcgtatctag accaatgaac acgaccttgg aagagatttt ttttgacagc    1140
aatctgggtt caaagaaact gacatcgact tgggatatct acgacctatg ggccaacaga    1200
gttgacaact cgacagcgtc tgctatcctt ggacggaata agacagccac cggtattctc    1260
tacaatgcta cggagcaatc ctacaaagac ggtttgtcta agaatgatac aagactgttt    1320
ggtcagaaaa ttggtagtct ttctccaaat gctatactta acacgactgt tccagctcac    1380
ggtatcgcct tctataggtt gagaccctct tcttgagctt attgttgagc aaagcagggc    1440
gagaagtatt gatgattg                                                  1458


<210>  5
<211>  54
<212>  DNA
<213>  Artificial sequence

<220>
<223>  Forward primer for amplifying the entire MEL1 gene

<400>  5
ctatatcgta atacaccaag ctcgacctcg cgatgtttgc tttctacttt ctca           54

<210>  6
<211>  52
<212>  DNA
<213>  Artificial sequence

<220>
<223>  Reverse primer for amplifying the entire MEL1 gene

<400>  6
ggtcgacggg cccggatcca tcgatagatc tcaatcatca atacttctcg cc             52


<210>  7

<211>  1362
<212>  DNA
<213>  Saccharomyces cerevisiae

<400>  7
gtgtctccga gttacaatgg ccttggtctc actccacaga tgggttggga caactggaat      60
acgtttgcct gcgatgtcag tgaacagcta cttctagaca ccgctgatag aatttctgac     120
ttggggctaa aggatatggg ttacaagtat atcattctgg atgactgctg gtctagcggc     180
agagattccg acggtttcct cgttgcagat gaacaaaaat ttcccaatgg tatgggccat     240
gttgcagacc acctgcataa taacagcttt cttttcggta tgtattcgtc tgctggtgag     300
tacacctgtg ctggatatcc tgggtctctg ggtcgtgagg aagaagatgc acagttcttt     360
gcaaataacc gcgttgacta cttgaagtac gataattgtt acaataaggg tcagtttggt     420
acaccggaaa tttcttacca ccgttacaag gccatgtcag atgctttgaa taaaactggt     480
aggcctatat tctattctct atgtaactgg ggtcaggatt taacatttta ctggggctct     540
ggtatcgcca attcttggag aatgagtgga gatgttactg ctgagttcac tcgtccagat     600
agcagatgtc cctgtgatgg cgatgaatac gattgcaagt acgccggttt ccattgttct     660
attatgaata ttcttaacaa ggcagctcca atggggcaaa atgcaggtgt tggtggttgg     720
aatgatctgg acaatctaga ggttggtgtc gggaatttga ctgacgatga ggaaaaggca     780
catttctcta tgtgggcaat ggtaaagtct ccacttatca ttggtgccaa tgtgaataac     840
ttaaaggcat cttcgtactc aatctatagt caagcctctg tcatcgcaat taatcaagat     900
tcaaatggta ttccagcaac aagagtctgg agatattatg tttcagacac agatgaatat     960
ggacaaggtg aaattcaaat gtggagtggt cctcttgaca atggtgatca agtggttgct    1020
ttattgaatg gaggaagcgt atctagacca atgaacacga ccttggaaga gatttttttt    1080
gacagcaatc tgggttcaaa gaaactgaca tcgacttggg atatctacga cctatgggcc    1140
aacagagttg acaactcgac agcgtctgct atccttggac ggaataagac agccaccggt    1200
attctctaca atgctacgga gcaatcctac aaagacggtt tgtctaagaa tgatacaaga    1260
ctgtttggtc agaaaattgg tagtctttct ccaaatgcta tacttaacac gactgttcca    1320
gctcacggta tcgccttcta taggttgaga ccctcttctt ga                        1362

<210>  8
<211>  51
<212>  DNA
<213>  Artificial sequence

<220>
<223>  Forward primer for amplifying MEL1 without secretion signal

<400>  8
cccgggagat ctatcgatgg atccgggccc gtgtctccga gttacaatgg c               51

<210>  9
<211>  51
<212>  DNA
<213>  Saccharomyces cerevisiae

<400>  9
tgggacgctc gacggatcag cggccgctta tcaagaagag ggtctcaacc t               51

<210>  10
<211>  243
<212>  DNA
<213>  Saccharomyces cerevisiae

<400>  10
tcgagcgtcc caaaaccttc tcaagcaagg ttttcagtat aatgttacat gcgtacacgc      60
gtttgtacag aaaaaaaaga aaaatttgaa atataaataa cgttcttaat actaacataa     120
ctattaaaaa aaataaatag ggacctagac ttcaggttgt ctaactcctt ccttttcggt     180
tagagcggat gtgggaggag ggcgtgaatg taagcgtgac ataactaatt acatgatatc     240
gac                                                                   243

1

## Description

### Field of the Invention

The invention relates to strains of S. *cerevisiae* which are capable of producing α-galactosidase. For better secretion thereof, the gene of α-galactosidase is fused to a signal sequence. Said strains are useful in methods for producing α-galactosidase, biomass and ethanol from a medium rich in raffinose, melibiose and/or stachyose and, in the event that the strains contain a second construct which expresses a protein of therapeutic interest, for producing said therapeutic proteins.

### Background of the Invention

α-galactosidases (EC 3.2.1.22) catalyze the hydrolysis of galactose residues bound by α(1,6) bonds of galacto-oligosaccharides and galacto-mannan polymers (mannose polymers with galactose branches), as well as the hydrolysis of oligosaccharides such as stachyose, raffinose and melibiose present in beans, soy beans and other legumes.

These enzymes are widely distributed in animals, plants and microorganisms. However, most monogastric mammals, including humans, lack pancreatic α-galactosidase such that this type of oligosaccharides cannot be digested in the digestive system and are fermented by the intestinal microflora producing gases which cause flatulence and other types of gastrointestinal disorders which may lead to problems with varying severity in sensitive individuals. Soy beans and the products derived therefrom have particular importance as they are an important component in the diet of many people given that they are a good source of proteins and are used in some cases as a dairy product substitutes, for example in people with lactose intolerance. There are food supplements consisting of α-galactosidase concentrates from different origins (*Lactococus, Aspergillus niger, Saccharomyces,* etc.), such as, for example, the tablets manufactured by Promefarm (Sinaire), for treating these disorders. The pre-treatment of these soy bean products as well as of other products prepared for human and animal consumption with a high content of non-digestible oligosaccharides such as raffinose and stachyose with α-galactosidase is a good alternative to avoid these gastrointestinal problems. It further contributes to increasing the nutritional value thereof; this point is generally very important in animal nutrition because the exploitation of energy from feed increases.

In humans, α-galactosidase is a lysosomal exoglycosidase which acts on α-galactosyl-type terminal residues of glycolipids and glycoproteins. Mutations in this gene cause deficiencies in these degradations which result in Fabry disease (X-linked lysosomal storage disease). This disease has an incidence of 1/40,000. Today there are treatments, such as the one developed by Genzyme Corp. (Fabrazyme), based on enzyme replacement technique for treating this disease by means of using human α-galactosidase expressed in CHO mice. A more cost-effective alternative is to use mammal cells for the heterologous production of the protein; the use of human α-galactosidase recombinantly expressed in yeasts has been studied.

In addition, the glycoprotein calyx on the surface of red blood cells determines, among many other functions, the blood type of each individual. Type O blood can be transfused to any individual, therefore it is the most needed. Type O blood can be imitated from type-B blood by using α-galactosidases capable of processing α(1-3)-type galactose bonds.

α-galactosidase is also used in the production of caramel and sugar at the industrial level to remove the presence of raffinose which prevents the proper crystallization of caramel, and to thus improve the. Furthermore, molasses, a waste product of this industry, has a high raffinose and stachyose content which means that it cannot be discarded directly as it has a high biodegradability (BOD). This enzyme or organisms producing it are used to degrade these sugars and to couple this degradation to another function, such as the production of ethanol or biomass. Further, molasses are the most widely used substrate for producing the nearly 430000 tons of S. *cerevisiae* for making bread (dry weight) that are produced every year. The strains of yeasts used in making bread lack α-galactosidase and strains of *Saccharomyces* have been developed for making bread with the gene encoding the α-galactosidase of S. *cerevisiae* var. *uvarum* for the purpose of improving the production of this biomass (Liljeström-Suominen et al.; Appl Environ Microbiol. 1988 Jan; 54(1):245-249).

In the case of yeasts, it has been described that α-galactosidase releases the non-reducing residues in the terminal position of galactose located at terminal ends of the substrates but not the internal residues (). In addition to the hydrolytic activity, α-galactosidases have the capacity to synthesize oligosaccharides by transglycosylation and reverse hydrolysis in the presence of high concentrations of monosaccharides (). *Saccharomyces cerevisiae* is one of the most studied sources of α-galactosidase and its use in biotechnological applications is extensive ().

Given the importance and the extensive biotechnological applications of this enzyme, there are data concerning the production of α-galactosidase from bacteria such as *Bacillus stearothermophilus* (US patent no. 3846239), *Thermus* sp. strain T2 (ES patent no. 2172380), *Streptomyces griseoloalbus* (Anisha et al.; Appl Biochem Biotechnol. 2008 Sept 4), however, and given that many of the applications that this enzyme will have will be related to the processing or preparation of materials related to either animal or human food, it is essential that the microorganism which will be used is a GRAS (Generally Recognized As Safe) microorganism, as is the case of S. *cerevisiae.* There are also data concerning the production of α-galactosidase from strains of fungus from the *Neurospora* and Rhizopus (Worthington and Beuchat, J Agric Food Chem. 1974; 22(6):1063-6), *Aspergillus oryzae, A. foetidus* (Liu et al., Lett Appl Microbiol. 2007 Aug;45(2):206-12), A. *ficuum* (Zapater et al., Prep Biochem. 1990;20(3-4):263-96) and A. niger (US patent nos. 6197566 and 5919690) genera, among others, although one of the main drawbacks of the production in fungi is the production of unwanted by-products. In the case of the production of α-galactosidase in A. niger, significant amounts of oxalic acid are produced. Likewise, there are data on the production of non-human α-galactosidase, such as from the guar plant (*Cyamopsis tetragonoloba*) fused to different signal sequences (Harmsen, M. et al., Gene. 1993 Mar 30; 125(2):115-23) exist.

With regard to the production in yeasts, US patent 4431737 describes a mutated α-galactosidase-producing strain of S. cerevisiae obtained by mutagenesis by means of ultraviolet radiations. The drawback of the strains mutated by means of a mutagenic treatment with ultraviolet radiation is that this treatment fundamentally alters the DNA due to the formation of pyrimidine primers causing a local distortion of the double helix configuration, which interferes in normal complementary base pairing; this in turn interferes in the replication and transcription processes and subsequently in growth and respiration. Therefore, in addition to being able to have other unwanted mutations, the strains thus mutated also usually have slow growth rates. There is another patent (US no. 5055401) in which strains of *Saccharomyces* transformed with the gene encoding α-galactosidase of *S. cerevisiae* var. *uvarum* (Liljeström-Suominen et al., 1988) has been constructed. Human α-galactosidase secreted into the medium from other yeasts, such as *Pichia Pastoris* (Chen, Y. et al., Protein Expr Purif. 2000 Dec; 20(3):472-84), has also been produced.

Therefore, producing and obtaining α-galactosidase from yeasts in an efficient and improved manner is an objective of especial interest in biotechnology.

### Summary of the Invention

The present invention provides strains of S. *cerevisiae* transformed with the constructs of the invention, α-galactosidase generated from yeasts with multiple applications in the food industry and biotechnology companies being produced. The exploitation of the catalytic activity of α-galactosidase is of interest for example in the hydrolysis of raffinose during the production of beet sugar and in soy bean processing; in the use of molasses by the strains of yeast, in the development of treatments for Fabry disease and as a food supplement to maximize the exploitation of energy in animal diets and to treat gastrointestinal disorders in humans.

Therefore, in one aspect, the invention relates to a DNA construct comprising a heterologous promoter regulated by means of glucose repression, a sequence encoding a functional signal sequence in yeast, and the region of the MEL1 gene encoding the mature form of α-galactosidase or a functionally equivalent variant thereof; wherein sequences (b) and (c) must be under the same reading frame.

In another aspect, the invention relates to a DNA construct comprising the ADH2 promoter or a functionally equivalent variant thereof, a sequence encoding a functional signal sequence in yeast and the region of a gene encoding the mature form of α-galactosidase or a functionally equivalent variant thereof; wherein the signal sequence and the gene encoding a mature form of α-galactosidase must be under the same reading frame.

In another aspect, the invention relates to a protein encoded by one of the DNA constructs of the invention.

In another aspect, the invention relates to an expression vector containing the construct of the invention.

In yet another aspect, the invention relates to a microorganism containing the constructs of the invention, the protein encoded by one of the DNA constructs of the invention, the expression vector with one of the constructs of the invention.

In another aspect, the invention relates to a method for producing α-galactosidase comprising the steps of culturing a microorganism and recovering the secreted α-galactosidase from the culture medium.

In another aspect, the invention relates to a method for obtaining cell biomass comprising the steps of culturing a microorganism containing the expression vector with the construct of the invention in a substrate containing non-reducing residues of α-D-galactose in the terminal position and recovering the cell biomass.

In another aspect, the invention relates to a method for obtaining ethanol comprising the steps of culturing a microorganism containing the expression vector with the construct of the invention in a substrate containing non-reducing residues of α-D-galactose in the terminal position and recovering the ethanol produced.

In another aspect, the invention relates to a microorganism containing the expression vector with the construct of the invention and furthermore a second construct with a gene encoding a protein of interest.

In another aspect, the invention relates to a method for obtaining a protein comprising the steps of culturing a microorganism containing the expression vector with the construct of the invention and furthermore a second construct with a gene encoding a protein of interest, in a substrate containing non-reducing residues of α-D-galactose in the terminal position and recovering the protein of interest.

### Brief Descriptions of the Drawings

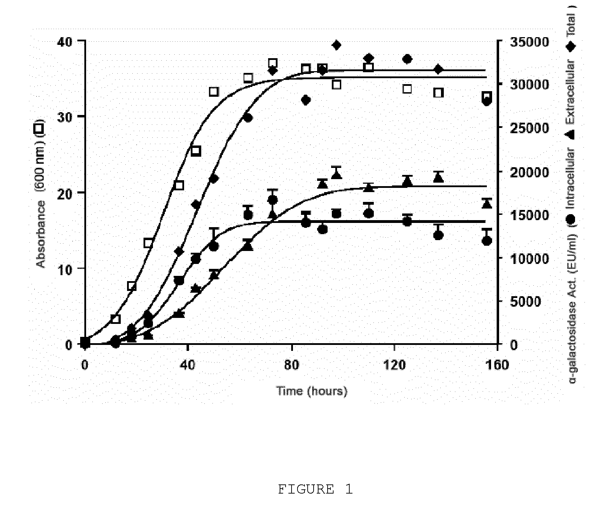

Figure 1 shows the absorbance measurements at 600 nm and the extracellular, intracellular and total α-galactosidase activity curves of the strain of S. *cerevisiae* transformed with the secretion plasmid containing the entire gene which encodes α-galactosidase of S. *cerevisiae.* The measurements of extracellular and intracellular α-galactosidase activity are the result of four independent measurements.

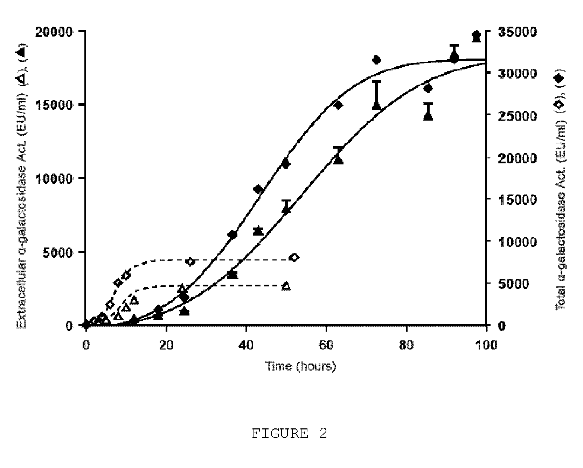

Figure 2 shows the measurements comparing the extracellular and total α-galactosidase activity of the strain of S. *cerevisiae* transformed with the secretion plasmid containing the entire gene which encodes α-galactosidase of S. *cerevisiae* (black symbols and solid lines) with the strain of S. *cerevisiae* transformed with the gene which encodes α-galactosidase of S. *cerevisiae* under the ADH1 promoter (as used in US 5055401) (white symbols and dotted lines).

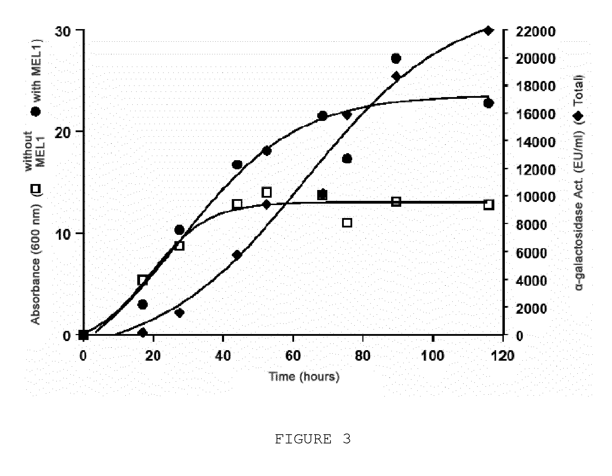

Figure 3 shows the absorbance measurements at 600 nm and the total α-galactosidase activity curves of the strain of S. *cerevisiae* transformed with the secretion plasmid containing the entire gene which encodes α-galactosidase of S. *cerevisiae* growing in a synthetic medium with 2% raffinose. The absorbance values are compared at 600 nm with those of the same strain but not transformed with the MEL1 gene.

### Detailed Description of the Invention

The authors of the present invention have developed a DNA construct which is made up of a heterologous promoter regulated by means of glucose repression, a sequence encoding a functional signal sequence in yeast fused in the same reading frame with a gene encoding the mature form of α-galactosidase of S. *cerevisiae.* They have shown that the strains of yeast containing said construct secrete α-galactosidase into the medium in its active form, which allows the use of the strains for multiple applications in the food industry and biotechnology companies. As a result of these features, the strains provide a solution to the problems existing in the prior art.

Thus, in a first aspect, the invention relates to a DNA construct comprising:
(a) a heterologous promoter regulated by means of glucose repression,
(b) a sequence encoding a functional signal sequence in yeast, and
(c) the region of the MEL1 gene encoding the mature form of α-galactosidase or a functionally equivalent variant thereof, wherein sequences (b) and (c) must be under the same reading frame. It will be hereinafter referred to as first construct of the invention.

The component (a) of the first construct of the invention is a heterologous promoter regulated by means of glucose repression. The term "heterologous promoter regulated by means of glucose repression" refers to the gene of interest being regulated by a promoter of a gene different from the gene of interest and on the other hand, it refers to promoters the expression of which is subject to glucose repression such that the expression of α-galactosidase will be repressed when the concentration of glucose in the medium starts to increase.

Promoters useful for carrying out the present invention include promoters such as the S. *cerevisiae* glyceraldehyde-3-phosphate (GAP) dehydrogenase promoter, the galactokinase (GAL1 and GAL7) promoter and the 3-phosphoglycerate kinase (PGK) gene promoter. In a preferred embodiment, the promoter is the ADH2 gene promoter, the sequence ID of which is SEQ ID N0:1.

The component (b) of the first construct of the invention is a sequence encoding a functional signal sequence in yeast.

"Functional signal sequence in yeast" refers to a peptide capable of promoting the secretion of any protein into the extracellular medium, in a particular embodiment, the protein is α-galactosidase.

Non-limiting illustrative examples of signal sequences which can be used in the context of the present invention include the signal sequence of α factor of *S. cerevisiae* and of other species from the *Kluyveromyces, Pichia,* and *Hansenula* genera, the signal sequence of the killer toxin of *K. lactis,* the signal sequence of glucoamylase II of S. *diastaticus,* the signal sequence of the glucoamylase of C. *albicans,* the signal sequence of the phosphatase of *S.cerevisiae,* the signal sequence of the 128 kDa killer toxin of *S.cerevisiae,* the signal sequence of the invertase of S. *cerevisiae,* as well as random sequences which are known for their capacity to functionally replace native signal sequences of *E.coli,* as described by Kaiser,C. et al. (Science, 1987, 235:312-317) and signal sequences which can be identified using the methods known in the art (for example by Gallicioti,G. et al. J. Membrane Biology, 183:175-182).

The signal sequence and α-galactosidase can be bound directly or they can alternatively be separated by a spacer peptide which is bound to the α-galactosidase after processing the signal sequence and which serves for the identification of α-galactosidase or for the purification thereof from the culture medium. In the first case, virtually any peptide or peptide sequence which allows the recognition of the peptide or fusion protein can be used, for example a peptide sequence recognized by a monoclonal antibody and which can serve to recognize the resulting fusion protein by immunoaffinity chromatography, for example tag peptides such as c-myc, HA, E, FLAG and generally any other sequence recognized by an antibody. In the second case, virtually any peptide or peptide sequence which allows isolating or purifying the peptide or fusion protein can be used, for example a polyhistidine sequence, a peptide sequence recognized by a monoclonal antibody and which can serve to purify the resulting fusion protein by immunoaffinity chromatography, for example tag peptides such as c-myc, HA, E, FLAG, etc. [Using Antibodies: A laboratory manual. Ed Harlow and David Lane (1999). Cold Spring Harbor Laboratory Press. New Cork. Chapter: Tagging proteins. pp. 347-377] and generally any other sequence recognized by an antibody. The tag peptide is preferably the FLAG epitope (SEQ ID NO:2), and it is connected to the C-terminus of the signal sequence and to the N-terminus of α-galactosidase, such that after removing the signal sequence, the FLAG epitope forms the N-terminus of the α-galactosidase.

In a preferred embodiment, the DNA construct can comprise a sequence encoding the sequence of α factor of S. *cerevisiae* (SEQ ID NO:3) or the endogenous signal sequence of the MEL1 gene of *S. cerevisiae,* fused through its 3' end and in the same reading frame with the sequence encoding the α-galactosidase.

The third element (c) of the first DNA construct of the invention is the region of the MEL1 gene encoding the mature form of α-galactosidase (SEQ ID NO:4) protein or a functionally equivalent variant thereof.

Functionally equivalent variant of the sequence identified as SEQ ID NO:4 is understood as any other sequence resulting from the insertion, deletion or substitution of one or more amino acids which maintains the α-galactosidase activity.

Methods for determining the α-galactosidase activity are sufficiently known in the prior art. Said methods are based on determining the capacity of the enzyme to hydrolyze a chromogenic, fluorogenic or chemiluminescent conjugate having galactose conjugated to a chromophore/fluoride/chemiluminescent compound by means of an α-glycosidic bond where the release of said compound can be detected by means of measuring the fluorescence released, the optical density at a determined wavelength or the chemiluminescence. Chromogenic compounds that can be used as substrates of the α-galactosidase to determine its activity include p-nitrophenyl-α-D-galactopyranoside (PNPG) following the method described by Kew and Douglas (Kew, O. M. and Douglas, H.C., 1976) which, upon being hydrolyzed, produce an intense yellow compound generated from the change of pH occurring once the enzymatic reaction stops and which is proportional to the amount of substrate released.

Other known α-galactosidase activity assays are those described by Dey and Pridham, 1969 (Dey, P.M., Pridham, J.B., 1969. Biochem. J. 113, 49-55); Lazo et al., 1977 (Lazo, P. S., Ochoa, A. G., Gascon, S., 1977. Eur. J. Biochem. 77(2): 375-382); Ryan et al., 1998 (Ryan, M.P., Jones, R., Morse, R.H., 1998. Mol Cell Biol 18(4): 1774-82); Garroa et al., 2004 (Garroa MS, Valdeza GF, Gioria GS., 2004. Food Microbiol 21:511-8.); Liu et al., 2007 (Liu, C., Ruan, HOURS., Shen, HOURS., Chen, Q., Zhou, B., Li, Y., He, G., 2007. J Food Sci 72(4): M120-M125).

Suitable conditions for performing the α-galactosidase assay are widely known in the prior art, for example, in Maniatis et al. (Molecular Cloning: A Laboratory Manual, Cold Spring Harbor Laboratory, Cold Spring Harbor, NY (1982)).

In a second aspect, the invention relates to a DNA construct comprising:
(a) the ADH2 promoter or a functionally equivalent variant thereof,
(b) a sequence encoding a functional signal sequence in yeast, and
(c) the region of a gene encoding a mature form of α-galactosidase or a functionally equivalent variant thereof, wherein sequences (b) and (c) must be under the same reading frame.

The ADH2 promoter is the alcohol dehydrogenase II gene promoter and is subject to catabolite repression.

The term "functional signal sequence in yeast" is defined in the first aspect and applies in the same manner in this case. In a preferred embodiment, the signal sequence is the endogenous signal sequence of the MEL1 gene or the signal sequence of the yeast α, factor.

A mature form of α-galactosidase may correspond to any α-galactosidase present in animals, plants and microorganisms. The same criteria are applied for the term "functionally equivalent variant" as in the first aspect. A preferred embodiment would be one in which the gene encoding the α-galactosidase protein is the MEL1 gene.

In addition, in another aspect, the invention relates to a protein encoded by the previously defined constructs and to an expression vector containing said constructs.

The protein generated from one of the DNA constructs of the invention comprises a functional signal sequence in yeast and a α-galactosidase, fused together such that the C-terminus of the signal sequence is fused with the N-terminus of the α-galactosidase.

The term "expression vector" refers to a replicative DNA construct used for expressing DNA which encodes the polypeptide of the invention or the fusion protein of the invention and which includes a transcriptional unit comprising the assembly of (1) genetic element(s) which play a regulatory role in gene expression, for example promoters, operators or enhancers, operatively bound to (2) a DNA sequence encoding the polypeptide or the fusion protein of the invention which is transcribed into messenger RNA and translated into protein and (3) suitable sequences to initiate and to terminate the transcription and translation.

The vectors that can be used in the context of the present invention normally include a genetic marker, a replication origin in bacteria or yeasts, multiple cloning sites, and a genetic marker. The genetic marker is usually a gene conferring resistance to an antibiotic or alternatively, an auxotrophic marker in the case of yeasts.

The yeast vectors suitable for the present invention can be based on the following types of plasmids
- Multicopy autonomous plasmids: These plasmids contain sequences which allow generating multiple copies of said vectors. These sequences can be the so-called 2µ such as the one which appears in episomal plasmids (YEp or yeast episomal plasmids) or ARS-type sequences such as those which appear in replication plasmids (YRps or yeast replication plasmids). Examples of vectors based on plasmids of this type are p426GPD, p416GPD, p426TEF, p423GPD, p425GPD, p424GPD or p426GAL, YEp24 and YEplac.
- Single copy autonomous plasmids: Plasmids which contain the autonomous replication sequence ARS1 and a centromere sequence (CEN4). Plasmids of this type include the centromere plasmids (YCps or yeast centromere plasmids).
- Integrating plasmids: Plasmids which are capable of being integrated into the host cell genome. Plasmids of this type include integrating plasmids (YIPs or yeast integrating plasmids). Examples of vectors based on plasmids of this type are pRS303, pRS304, pRS305 or pRS306 and the like.

Generally, all the vectors mentioned by Sikorski ("Extrachromosomal cloning vectors of Saccharomyces cerevisiae", in Plasmid, A Practical Approach, Ed. K. G. Hardy, IRL Press, 1993) and by Ausubel et al. ("Yeast Cloning Vectors and Genes" Current Protocols in Molecular Biology, Section II, Unit 13.4, 1994) are useful in the context of the present invention.

In another aspect, the invention relates to a microorganism containing the DNA construct according to the invention, the expression vector containing the construct of the invention or the fusion protein according to the invention. In a particular embodiment, the microorganism is a yeast. Yeast is understood as any eukaryotic organism belonging to the ascomycetes type which includes the organisms generally know as yeasts as well as those generally known as filamentous fungi. The yeasts and filamentous fungi include *Pichia* sp (for example, *P. pastoris, P. finlandica, P. trehalophila, P. koclamae, P. membranaefaciens, P. minuta, P. opuntiae, P. thermotolerans, P. salictaria, P. guercuum, P. pijperi, P. stiptis, P. methanolica), Saccharomyces (S. cerevisiae), Schizosaccharomyces pombe, Kluyveromyces* (for example, *K. lactis, K. fragilis, K. bulgaricus, K. Wickeramii, K. waltii, K. drosophilarum, K. thernotolerans, and K. marxianus, K. yarrowia), Trichoderma reesia, Neurospora crassa, Schwanniomyces, Schwanniomyces* occidentalis, Penicillium, *Totypocladium, Aspergillus* (for example, A. *nidulans,* A. niger, A. oryzae), *Hansenula polymorpha, Candida, Kloeckera, Torulopsis, and Rhodotorula, Hansenula, Kluyveromyces sp.* (for example, *Kluyveromyces lactis), Candida albicans, Aspergillus sp* (for example, *Aspergillus nidulans, Aspergillum* niger, *Aspergillus oryzae*)*, Trichoderma reesei, Chrysosporium luchiowense, Fusarium sp.* (for example, *Fusarium gramineum, Fusarium venenatum), Physcomitrella patens.*

Virtually any yeast can be used to carry out the method of the invention; however, in a particular embodiment, said yeast is a yeast from the *Saccharomyces* genus, such as S. *cerevisiae.*

To obtain the yeast of the invention , it is necessary to introduce the vector containing the construct into the yeast cell. Methods suitable for introducing a DNA molecule into a yeast cell include:
- Transformation of spheroplasts which entails removing the cell wall of the yeast and contacting the spheroplasts with the plasmid in the presence of PEG.
- Transformation with Li⁺, which entails the treatment of yeast cells with monovalent alkaline cations (Na⁺, K⁺, Rb⁺, Cs⁺ and Li⁺) in combination with PEG to stimulate DNA uptake by the intact cells.
- Electroporation, which entails administering electrical pulses to the yeasts which results in the opening of pores in the membrane of the spheroplasts and intact yeast cells.

The microorganisms of the invention are capable of producing and secreting α-galactosidase into the medium. Thus, in another aspect, the invention relates to a method for producing α-galactosidase which comprises culturing a microorganism according to the invention and recovering the secreted α-galactosidase from the culture medium.

The α-galactosidase protein can be easily purified in a single affinity chromatography step by means of using substrate analogs such as, for example, p-aminophenyl-β-D-thiogalactopyranoside (Steers, E. et al, 1970, J.Biol.Chem. 246:196-200).

The determination of the degree of purity of the α-galactosidase can be estimated by means of the value of the specific enzymatic activity calculated by dividing the number of units of enzymatic activity by the amount of protein, in mg, in a determined volume. Preferably, the enzymatic activity is determined by means of the Guarente, L. method (Methods Enzymol. 1983, 101:181-191).

The microorganisms of the invention are capable of growing in media containing a substrate rich in non-reducing residues of α-D-galactose in the terminal position as the sole carbon source. Thus, in another aspect, the invention relates to a method for obtaining cell biomass from a composition rich in residues of non-reducing α-D-galactose in the terminal position which comprises (a) culturing a microorganism comprising one of the constructs of the invention and (b) recovering the biomass from the culture medium.

The preferred media rich in residues of non-reducing α-D-galactose in the terminal position are media rich in stachyose, raffinose, melibiose, sugar cane and/or beet molasses, soy protein permeates and a combination of any of the foregoing.

The biomass can be recovered from the culture medium by means of any technique known by the person skilled in the art including, but not limited, to centrifugation, deposition or filtration. The technique used must preferably minimize cell damage to the maximum. In the event that the same culture is used for preparing biomass and producing α-galactosidase, the separation of the cell biomass must minimize the loss of α-galactosidase to the maximum.

The microorganism recovered from the culture medium is typically washed with an aqueous solution to remove unwanted materials that may be associated therewith. The protein content in the yeast is preferably between 35 and 65%. The recovered yeast biomass can be used as an ingredient in food products without the need for additional processing. The recovered biomass can also be lysed and, optionally, the intact cells separated. The lysed yeast cells can be used in culture media as a yeast extract or they can be additionally processed to separate their different components such as peptides, nucleotides, amino acids or specific components of the cell wall, such as chitin, glucans, mannans and oligosaccharides.

The substrates susceptible to being used (stachyose, raffinose, melibiose, sugar cane and/or beet molasses and/or soy protein permeates) are digested by the yeast of the invention, generating galactose which would be a suitable substrate for alcohol fermentation, ethanol or other compounds being produced. Thus, in another aspect, the invention relates to a method for obtaining a fermentation product comprising the steps of culturing a microorganism in a medium rich in non-reducing residues of α-D-galactose in the terminal position, wherein said yeast comprising a DNA construct encoding a fusion protein with the mature form of α-galactosidase fused in the same reading frame with a signal sequence, wherein said signal sequence is capable of promoting the secretion of α-galactosidase of a yeast cell and (b) recovering the fermentation product from the culture medium.

Fermentation product is understood in the context of the present invention as a product which can be obtained from a yeast from a sugar in anaerobic conditions. Fermentation products which can be obtained using the strains and methods of the present invention include alcohols (ethanol, methanol, butanol), organic acids (for example, citric acid, acetic acid, itaconic acid), ketones (for example, acetone), amino acids (for example, glutamic acid), gases (H₂ and CO₂), antibiotics (penicillin, tetracycline), etc. In a preferred embodiment, the fermentation product is ethanol which can be used as fuel, in drinks or at an industrial level. The alcohol fermentation for giving rise to ethanol is typically carried out for 30-60 hours at a temperature around 32°C.

The culture conditions for producing fermentation products are essentially the same as those used in the production of α-galactosidase in terms of the culture media that can be used as a carbon source. Nevertheless, for the purpose of improving the yield of the reaction, it is important for the culture conditions to be those that favor monosaccharide fermentation. Once the suitable concentration of fermentation product has been reached in the medium, it is necessary to recover said product from the medium. The way to recover the product will depend on the chemical nature of the product. In the event that the fermentation product is ethanol, it is recovered using conventional techniques which include distillation, for example.

The microorganisms of the present invention can also be transformed with a second expression vector containing a gene encoding a protein of interest and thus be used to produce proteins of interest by means of culturing said doubly transformed strains in a culture medium rich in non-reducing residues of α-D-galactose in the terminal position as a single carbon source.

Thus in another aspect, the invention relates to a microorganism comprising in addition to the first construct of the invention, a second construct with a gene encoding a protein of interest.

The expression vectors that can be used for expressing the heterologous proteins are essentially the same as those that can be used for expressing the fusion protein with α-galactosidase. However, it is advisable for the two vectors to have different selection markers to ensure that both of them remain stable in the yeasts. The promoters which regulate the expression of the heterologous protein can also be the same as those used to regulate the expression of the fusion proteins. Promoters useful for producing the present construct include:
- Constitutive promoters such as, for example, the alcohol dehydrogenase (ADH1) promoter, the 1-α, elongation factor (TEF) promoter and the promoter of the gene which encodes triose phosphate isomerase (TPI), the glyceraldehyde 3-phosphate dehydrogenase (GPD) promoter and the 3-phosphoglycerate kinase (GPK) promoter, the MRP7 promoter and the alcohol oxidase (AOX1) promoter.
- Inducible promoters such as, for example, the metallothionein (CUP1) promoter, the expression of which is regulated by means of adding copper to the culture medium, the promoter of the gene which encodes the FUS1 gene or the FUS2 gene, the expression of which is activated in the presence of pheromones (the α-factor) as described in US5063154, the TET promoter, the expression of which is regulated in the presence of tetracyclines, the GAL1-10, GALL, GALS promoters which are activated in the presence of galactose, the VP16-ER promoter, inducible by estrogens, and the phosphatase (PH05) promoter the expression of which is activated in the presence of phosphate and the HSP150 heat shock protein promoter, the expression of which is activated at a high temperature.
- Repressible promoters such as, for example, the *S.cerevisiae* enolase (ENO-1) gene promoter, the expression of which can be repressed when the microorganism is grown in a non-fermentable carbon source, as well as promoters the expression of which is subject to glucose repression such that the expression will be repressed when part of the lactose has been hydrolyzed and the concentration of glucose in the medium starts to increase, the S. *cerevisiae* glyceraldehyde-3-phosphate dehydrogenase (ADH2/GAP) promoter and the galactokinase (GALl) promoter.

Different promoters are preferably used in the two constructs such that the expression of the α-galactosidase and of the heterologous protein can be regulated independently. Preferably, in those cases in which the heterologous protein is suspected of being toxic to the host cell, the promoter used to regulate its expression is advisably a regulatable promoter such that the expression of the protein of interest can be delayed until sufficient biomass levels have been achieved. The invention contemplates the use of stachyose, raffinose, melibiose, sugar cane and/or beet molasses, soy protein permeates and a combination of any of the foregoing as the culture medium rich in non-reducing residues of α-D-galactose in the terminal position.

The proteins of interest may lack a signal sequence or, alternatively, they can be synthesized with a signal sequence for the purpose of provoking the release thereof into the culture medium and thus facilitating the recovery thereof. Signal sequences which can be used in the context of the present invention essentially include the same as those that can be used to promote the secretion of α-galactosidase.

The doubly transformed strains can be conveniently used for producing proteins of interest using non-reducing residues of α-D-galactose in the terminal position as the main carbon source. Thus, in another aspect, the invention relates to a method for obtaining a protein of interest comprising the steps of (a) culturing a doubly transformed strain of yeast according to the invention in a medium rich in non-reducing residues of α-D-galactose in the terminal position and (b) recovering the protein of interest from the medium.

In a preferred embodiment, the medium rich in non-reducing α-D-galactose residues in the terminal position is stachyose, melibiose, raffinose, sugar cane and/or beet molasses, soy protein permeates and a combination of any of the foregoing.

There is virtually no limit with respect to the protein of interest that can be expressed using the strains and methods of the present invention. These include both human and mammal proteins of therapeutic interest and enzymes of different origins which allow reconstituting metabolic pathways in yeast and the subsequent production of compounds of interest.

Examples of proteins of therapeutic interest that can be produced by the cells object of the invention are erythropoietin (EPO), adrenocorticotropic-releasing hormone (CRH), somatotropin-releasing hormone (SRH), gonadotropin-releasing hormone (GnRH), thyrotropin-releasing hormone (TRH), prolactin-releasing hormone (PRH), melanotropin-releasing hormone (MRH), prolactin-inhibiting hormone (PIH), somatostatin, adrenocorticotropic hormone (ACTH), somatroph or growth hormone (GH), luteinizing hormone (LH), follicle-stimulating hormone (FSH), thyrotropin (TSH or thyroid-stimulating hormone), prolactin, oxytocin, antidiuretic hormone (ADH or vasopressin), melatonin, Müllerian inhibiting factor, calcitonin, cholecystokinin hormone (CCK), secretin, insulin-like growth factor type I (IGF-I), insulin-like growth factor type II (IGF-II), atrial natriuretic peptide (ANP), human chorionic gonadotropin (hCG), insulin, glucagon, somatostatin, pancreatic polypeptide (PP), leptin, Y neuropeptide, renin, angiotensin I, angiotensin II, factor VIII, factor IX, tissue factor, factor VII, factor X, thrombin, factor V, factor XI, factor XIII, interleukin-1 (IL-1), tumor necrosis factor-alpha (TNF-α), interleukin-6 (IL-6), interleukin-8 (IL-8 and chemokines), interleukin-12 (IL-12), interleukin-16 (IL-16), α, β, gamma interferons, neuronal growth factor (NGF), platelet-derived growth factor (PDGF), transforming growth factor-β (TGF-β), bone morphogenetic proteins (BMPs), fibroblast growth factor (FGF and KGF), epidermal growth factor (EGF and the like), vascular endothelial growth factor (VEGF), granulocyte-colony stimulating factor (G-CSF), glial growth factor, keratinocyte growth factor, endothelial growth factor, α-1 antitrypsin, tumor necrosis factor, granulocyte and macrophage-colony stimulating factor (GM-CSF), cyclosporine, fibrinogen, lactoferrin, tissue-type plasminogen activator (tPA), chymotrypsin, immunoglobins, hirudin, superoxide dismutase and imiglucerase.

Virtually any method known in the prior art can be used for recovering the protein of interest from inside the cell or from culture medium. If the protein is produced inside the yeast cell, it is necessary to lyse the cells to release the proteins of interest. Yeast cells are conventionally lysed by means of hypotonic lysis of spheroplasts previously formed by means of treating with glucanases, by means of sonication or by means of shaking in the presence of glass beads. Once the protein of interest is in the medium either by means of being released from inside the cell or because the protein is secreted by the secretion machinery of the yeast itself, conventional methods are used for the purification of said protein, including, but not limiting, to chromatography (for example, ion exchange chromatography, affinity chromatography, hydrophobic interaction chromatography, gel filtration chromatography, HPLC), electrophoresis methods (preparative isoelectric focusing, preparative electrophoresis in polyacrylamide-SDS gels), differential solubility (precipitation with ammonium sulphate), preparative sucrose gradient ultracentrifugation. Once the desired degree of purity has been reached, which may require performing more than a single chromatography step, it is commonly necessary to concentrate the protein or to remove salts and ions which can be detrimental for subsequent use. In that case, known techniques such as lyophilization or ultrafiltration are used.

The invention is described below by means of non-limiting illustrative examples of the invention.

### Examples

### Example 1

### Obtaining the modified strain of yeast to produce α-galactosidase

### Materials and methods:

### 1. Cloning the nucleotide sequence from the gene encoding α-galactosidase

Two pairs of primers were designed for amplifying by PCR (Polymerase Chain Reaction) the MEL1 gene encoding the α-galactosidase of *Saccharomyces cerevisiae.* Two fragments were amplified, one of them corresponding to the complete gene of α-galactosidase (SEQ ID NO:4), amplified with the following primers, with SEQ ID NO:5 and SEQ ID NO:6 and the other fragment corresponding to the gene of the α-galactosidase in which the 54 nucleotides encoding the secretion signal (SEQ ID NO:7) were removed and amplified by the following primers, with sequences SEQ ID NO:8 and SEQ ID NO:9. The complete gene of the α-galactosidase was inserted into the vector YEpFLAG1 (Eastman Kodak Company Cat. No. IB13400) for expression in yeasts under the ADH2 (Alcohol Dehydrogenase 2) promoter (SEQ ID NO:1) and the transcription terminator of the CYC1 gene (SEQ ID NO:10). The second product of the amplification with the gene of the α-galactosidase without the secretion signal was inserted into a vector like the previous one, which further has the secretion signal of the yeast α factor (83 amino acids) and the FLAG peptide for its subsequent immunological detection (SEQ ID NO:2). The resulting constructs were transformed into a strain of *Saccharomyces cerevisiae (pep4::HIS3 prb-Δ1.6R HIS3 lys2-208 trp1*-Δ*101 ura3-52 gal2 can1*) by means of the lithium acetate method (Ito et al., J Bacteriol. 1983 Jan;153(1):163-8).

### 2. Culturing the recombinant strains

The strains transformed with the two constructs were cultivated in CM medium (Zitomer et al., J Biol Chem. 1976 Oct 25;251(20):6320-6), using tryptophan as an auxotrophic marker. When they reached the stationary growth phase, they were used to inoculate 100 ml of YPHSM medium (1% glucose, 3% glycerol, 1% yeast extract and 8% peptone) or to inoculate the same culture medium but said culture medium containing 2% raffinose instead of 1% glucose.

All the assayed cultures were grown at 30°C with orbital shaking at 250 r.p.m and starting from an initial absorbance at 600 nm of 0.05. The absorbance values at 600 nm, the extracellular and intracellular α-galactosidase activity and, depending on the case, consumption of raffinose, melibiose, fructose, glucose and galactose at different time intervals were determined.

In some cases, cultures were grown in 2 liters of medium in a Biostat-MD (Braun-Biotech)-type fermenter, monitoring the aeration (2 1/min), temperature (30°C), pH, shaking (250 r.p.m). Samples were collected at different time intervals and the aforementioned parameters were analyzed equally.

### 3. Determining the α-galactosidase activity

The measurements of the α-galactosidase activity *in vitro* were carried out using the chromogenic substrate p-nitrophenyl-α-D-galactopyranoside (PNPG) (*Sigma-Aldrich*) following the method described by Kew and Douglas (Kew, O. M. et al., J Bacteriol. 1976 Jan;125(1):33-41). The colorless PNPG compound gives rise to a product after hydrolysis which, due to the change of pH produced as the enzymatic reaction stops, acquires a yellowish color quantifiable by using a spectrophotometer and which is proportional to the amount of substrate released.

The α-galactosidase activity is expressed in enzymatic units; defining the enzymatic unit (E.U.) as the amount of enzyme which releases one nanomole of p-nitrophenyl per minute in the assay conditions (E.U.= nanomol x min⁻¹ x ml⁻¹). The units are expressed as E.U./ml of culture medium. 4. Determining the presence of raffinose, melibiose, fructose,

### glucose and galactose

A *Waters* HPLC (High Performance Liquid Chromatography) with isocratic pump model 1515 of the Breeze series with refraction index detector model 2414 and a Sugar Pak I separation column of 6.5 mm x 300 mm was used for the determination of raffinose, melibiose, fructose, glucose and galactose.

### Results

Figure 1 shows the cell growth obtained by means of measuring the absorbance at 600 nm and the extracellular and intracellular α-galactosidase activity of the strain of S. *cerevisiae* transformed with the expression plasmid containing the entire MEL1 gene encoding the α-galactosidase of S. *cerevisiae* growing in a synthetic medium with 1% glucose. The enzymatic activity data shown in Figure 1 is the result of 4 independent measurements. The total activity is the sum of the intracellular and extracellular activity. This recombinant strain secretes a mean of 18700 E.U./ml of α-galactosidase into the culture medium, between 90 and 140 hours, the mean total activity for the same time interval being 32000 E.U./ml, which means that the extracellular activity is 58.4% of the total activity.

For the purpose of verifying the growth of the recombinant strain with the complete MEL1 gene in media with raffinose, Figure 3 shows the absorbance measurements at 600 nm and the total α-galactosidase activity of the strain growing in a synthetic medium with 2% raffinose. By way of comparison, the absorbance measurements at 600 nm of the same non-transformed strain growing in 2% raffinose are shown. It can be observed that while the non-transformed strain reaches absorbance values of about 12 at the end of the culture, they are virtually doubled in the transformed strain, reaching values of about 22. The non-transformed strain can only use the fructose of raffinose but it is not capable of metabolizing melibiose. The transformed strain however, in addition to using the fructose of raffinose, is capable of using melibiose. The determination by means of HPLC of the raffinose, melibiose, fructose, glucose and galactose sugars confirmed the presence of melibiose in the culture in the non-transformed strain while in the transformed strain the melibiose virtually disappears after 48 hours of culture.

### Example 2

### Comparing the α-galactosidase activity of the strain with MEL1 under the ADH2 promoter [A] and of a strain with MEL1 under the ADH1 promoter [B].

The cloning details and the measurements of the α-galactosidase activity are described in Example 1. The culture of the recombinant strain [A] was carried out in a synthetic medium with 1% glucose.

Figure 2 shows a comparison of the data obtained by the strain [B] described in US 5055401 with the recombinant strain [A] of the invention. For that purpose, data have been extracted from Figures 7A and 7B of US 5055401 and they have been compared. It can be observed that while a total α-galactosidase activity of 8000 E.U./ml was reached at 36-54 hours in the strain described in US 5055401, a total activity of 10000 E.U./ml to 20000 E.U./ml were obtained for the same time interval in strain [A] of the invention, achieving, as previously discussed, values of about 32000 E.U./ml in the subsequent phases of the culture. Said increase involves an increment from 25% to 300% of the total α-galactosidase activity in the recombinant strain [A] of the invention with respect to the strain [B] described in US 5055401. Likewise, regarding the extracellular α-galactosidase activity of strain [B] described in US 5055401, when compared to the recombinant strain [A] of the invention, it can be observed that activity of about 2600 E.U./ml is obtained in the former at 36-54 hours, which is 32.5% of the total activity, while values of 3400 to 7800 E.U./ml (approximately 37.25% of the total activity) were obtained in strain [A] of the invention, increasing to 18700 E.U./ml (58.4% of the total activity) in subsequent phases of the culture.

## Claims

1. A DNA construct comprising:
(a) a heterologous promoter regulated by means of glucose repression,
(b) a sequence encoding a functional signal sequence in yeast, and
(c) the region of the MEL1 gene encoding the mature form of α-galactosidase or a functionally equivalent variant thereof, wherein sequences (b) and (c) must be under the same reading frame.

2. The construct according to claim 1, wherein the heterologous promoter regulated by means of glucose repression is the ADH2 promoter.

3. The construct according to claims 1 or 2, wherein the signal sequence is the endogenous signal sequence of the MEL1 gene.

4. The construct according to claims 1 or 2, wherein the signal sequence is the signal sequence of the yeast α factor.

5. A DNA construct comprising:
(a) the ADH2 promoter or a functionally equivalent variant thereof,
(b) a sequence encoding a functional signal sequence in yeast, and
(c) the region of a gene encoding a mature form of α-galactosidase or a functionally equivalent variant thereof, wherein sequences (b) and (c) must be under the same reading frame.

6. The construct according to claim 5, wherein the gene encoding the α-galactosidase protein is the MEL1 gene.

7. The construct according to claim 5 or 6, wherein the signal sequence is the endogenous signal sequence of the MEL1 gene.

8. The construct according to any of claims 5 to 7, wherein the signal sequence is the signal sequence of the yeast α factor.

9. A protein encoded by a construct according to any of claims 1 to 8.

10. An expression vector containing a construct according to any of claims 1 to 8.

11. A microorganism containing a vector according to claim 10, a DNA construct according to any of claims 1 to 8 or a protein according to claim 9.

12. The microorganism according to claim 11, wherein the microorganism is a yeast.

13. The microorganism according to claim 12, wherein the microorganism is *Saccharomyces cerevisiae.*

14. A method for producing α-galactosidase which comprises
(a) culturing a microorganism according to any of claims 11, 12 or 13 in a substrate containing non-reducing residues of α-D-galactose in the terminal position and
(b) recovering the secreted α-galactosidase from the culture medium.

15. A method for obtaining cell biomass which comprises
(a) culturing a microorganism according to any of claims 11, 12 or 13 in a substrate containing non-reducing residues of α-D-galactose in the terminal position and
(b) recovering the cell biomass.

16. A method for obtaining ethanol which comprises
(a) culturing a microorganism according to any of claims 11, 12 or 13 in a substrate containing non-reducing residues of α-D-galactose in the terminal position and
(b) recovering the ethanol produced.

17. A microorganism according to any of claims 11, 12 or 13, comprising a second construct with a gene encoding a protein of interest.

18. A method for obtaining a protein which comprises
(a) culturing a microorganism according to claim 17 in a substrate containing non-reducing residues of α-D-galactose in the terminal position and
(b) recovering the protein of interest.

19. The method according to any of claims 14 to 18, wherein the substrate containing non-reducing residues of α-D-galactose in the terminal position is selected from the group of raffinose, melibiose, stachyose, sugar cane and/or beet molasses, soy protein permeates and a combination of any of the foregoing.
